# EUROPÄISCHE PATENTSCHRIFT

(11) **EP 4 125 765 B1**
(45) Veröffentlichungstag und Bekanntmachung des Hinweises auf die Patenterteilung: **05.03.2025**
(21) Anmeldenummer: 20841874.9
(22) Anmeldetag: 14.12.2020
(51) Int. Cl.: A61G 13/12, A61F 5/37

(54) **FUSSROTATIONSVORRICHTUNG**
FOOT ROTATION DEVICE
DISPOSITIF DE ROTATION DE PIED

(30) Priorität: 23.03.2020 DE 102020107943
(43) Veröffentlichungstag der Anmeldung: 08.02.2023
(73) Patentinhaber: Condor Medtec GmbH, 33154 Salzkotten (DE)
(72) Erfinder: SCHULTE, Dominik, 33154 Salzkotten (DE); FECKE-SCHULTE, Ira, 33129 Delbrück (DE)
(74) Vertreter: Konrad, Stephan
(86) Internationale Anmeldenummer: PCT/DE2020/101057
(87) Internationale Veröffentlichungsnummer: WO 2021/190678

(56) Entgegenhaltungen:
- DE-U1- 202016 101 587
- US-A- 1 823 248
- US-A- 2 067 891
- US-A- 4 913 413
- US-A- 5 918 330

## Beschreibung

Die Erfindung betrifft eine Fußrotationsvorrichtung eines OP-Tisches gemäß dem Oberbegriff des ersten Patentanspruches.

Es ist eine Fußrotationsvorrichtung bekannt, US 2017/0056234 A1, die mit einer an einem verschwenkbaren Ausleger angeordneten Fußrotation mit einer Fußaufnahmeschale mit einem fersenseitigen Befestigungsblock und einem sich davon in einen mit einer Arretiervorrichtung versehenden Rotationsblock erstreckenden Fortsatz ausgestattet ist.

Der Rotationsblock gibt dem Anwender die Möglichkeit den Patientenfuß, bzw. das Patientenbein während einer Operation um seine Längsachse zu verdrehen und in einer gewünschten Position zu fixieren. Bei der bekannten Fußrotationsvorrichtung wird dies durch eine Klemmschraube bewirkt, die auf den Radius einer im Rotationsblock gelagerten Klemmkugel drückt.

Nachteilig an diesem bekannten Stand der Technik ist, dass die Winkelfixierung von der Spannkraft der Klemmschraube abhängig ist und somit von Anwender zu Anwender unterschiedlich und ggf. zu stark oder zu schwach ist, was bei einem stark verdrehten Patientenfuß bzw. Patientenbein und dadurch hoher vorliegender Torsionskraft zum Durchrutschen des Rotationsblockes führen kann. Ein weiterer Nachteil besteht darin, dass der Anwender, um während einer Operation die Torsion zu erhöhen, mit einer Hand die Klemmschraube lösen und mit der anderen den Patientenfuß und damit das Patientenbein verdrehen und halten muss, bis er die Klemmschraube wieder ausreichend fest angezogen hat. Nachteilig sind auch Situationen, bei denen ein starker Anwender eine Klemmschraube so stark angezogen hat, dass ein anderer Anwender sie nicht lösen kann, wodurch ein Operationsverlauf empfindlich gestört werden kann.

Eine solche Fußrotationsvorrichtung ist aus DE 20 2016 101 587 U1 bekannt.

Aufgabe der Erfindung ist es eine Fußrotationsvorrichtung an einem OP-Tisch zur Verfügung zu stellen, die unabhängig von manuellen Spannkräften in jeder gewünschten Position zu fixieren ist, wobei dies für jedermann leicht und betriebssicher durchführbar sein soll.

Die Lösung dieser Aufgabe ergibt sich in Verbindung mit den Merkmalen des Oberbegriffes des ersten Patentanspruches im Zusammenhang mit den technischen Merkmalen dessen kennzeichnenden Teils.

Die Rotation wird durch Sperrklinken des Zahngesperres gesperrt, die in mindestens ein Sperrzahnrad eingreifen, wobei die Winkelfixierung nach Vorwahl der Drehrichtung durch den formschlüssigen Eingriff eines oder mehrerer vorgespannt gehaltener Sperrhebel in ein oder mehrere Sperrzahnräder realisiert wird. Eine solche Rastung ist auch bei hohen Torsionskräften stets winkelstabil, wobei der Anwender dann, wenn während einer Operation die Torsion erhöht werden muss, lediglich mit einer Hand den Patientenfuß weiterdrehen muss, um das gesamte Patientenbein weiter zu tordieren. Die erneute Fixierung übernimmt das Sperrzahnrad anschließend automatisch, ohne dass eine Klemmschraube betätigt werden müsste, die reibschlüssig auf einer Klemmkugel aufliegt.

Weitere vorteilhafte Ausgestaltungen des Gegenstandes der Erfindung ergeben sich aus den nachfolgenden Unteransprüchen

Gemäß einer besonders bevorzugten Ausführungsform der Erfindung sind im Rotationsblock zwei Sperrzahnräder mit entgegengesetzt zueinander ausgebildeten radialen Sperrkerben geringfügig voneinander beabstandet auf einer gemeinsamen Welle angeordnet, die mit dem Fortsatz verbunden ist und zwischen die sich ein Schaltnocken des Betätigungsdrehgriffes erstreckt, wobei zwei Sperrhebel mit Sperrklinken um zur Drehachse der Welle parallele Achsen schwenkbar im Rotationsblock gelagert sind, die mit Steuernocken auf dem Schaltnocken des Betätigungsdrehgriffes aufliegen, wobei in Abhängigkeit dessen Verdrehwinkels mindestens eine Sperrklinke in ihr zugehöriges Sperrzahnrad eingreift oder davon abgehoben ist. Das Sperren oder die Freigabe der Drehbewegung in eine der beiden möglichen Richtungen erfolgt hier lediglich durch das einfache Umschalten des Drehgriffes, da dann in Abhängigkeit des Verdrehwinkels eine oder beide Sperrklinken in ihr zugehöriges Sperrzahnrad eingreifen oder davon abgehoben sind.

Besonders vorteilhaft ist eine Ausgestaltung der Erfindung, bei der sich der Schaltnocken zwischen die Sperrhebel erstreckt, die ihrerseits Steuernocken aufweisen, die durch eine Feder elastisch vorgespannt auf einer Nockenbahn des Schaltnockens aufliegen. Dieser weist eine kreiszylinderartige Grundform auf, wobei sich die Nockenbahn etwa über die Hälfte des Umfanges aus einem kreiszylinderartigen Abschnitt mit einem kleineren Durchmesser als dem der Grundform, eine Innenbahn bildend, erstreckt, an die sich beidseitig bis auf den größeren Durchmesser der Grundform geschwungene Kurvenbahnen anschließen, wobei die Nockenbahn von einem Teilstück mit dem Durchmesser der Grundform, eine Außenbahn bildend, vervollständigt wird.

In Verbindung mit dieser asymmetrischen Ausbildung der Kurvenbahn und der Anordnung der darauf ablaufenden Steuernocken der Sperrhebel lassen sich vier Schaltfunktionen verwirklichen, in denen die Steuernocken entweder gleichzeitig auf der Innenbahn oder gleichzeitig auf der Außenbahn anliegen oder aber abwechselnd einer auf der Außen- und einer auf der Innenbahn, wobei bei gleichzeitiger Lage auf der Innenbahn eine vollständige Sperrung der Rotation erfolgt, bei gleichzeitiger Anlage auf der Außenbahn ein vollständig beidseitiger Freilauf möglich ist oder bei einer um + oder - 90° dazu verdrehten Position des Betätigungsdrehgriffes mit einem Steuernocken auf der Innenbahn und einem Steuernocken auf der Außenbahn ein linksseitiger Freilauf und eine rechtseitige Sperrung oder ein rechtseitiger Freilauf mit linksseitiger Sperrung einstellbar ist.

Durch die erfinderische Anordnung der Nockenposition an den Sperrhebeln und dem von einem radialen Kontakt abweichenden Anordnung der Steuernocken auf der Nockenbahn des Schaltnockens werden demnach vier unterschiedliche Schaltfunktionen zur Verfügung gestellt.

Nachfolgend ist ein Ausführungsbeispiel der Erfindung anhand von Zeichnungen näher beschrieben. Es zeigen:

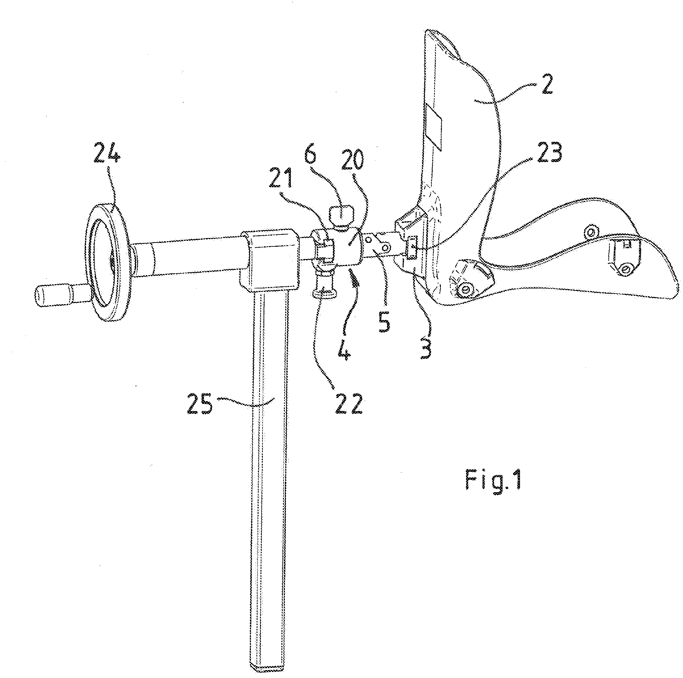

- Fig. 1: eine Fußrotationsvorrichtung eines OP-Tisches in räumlicher Ansicht,

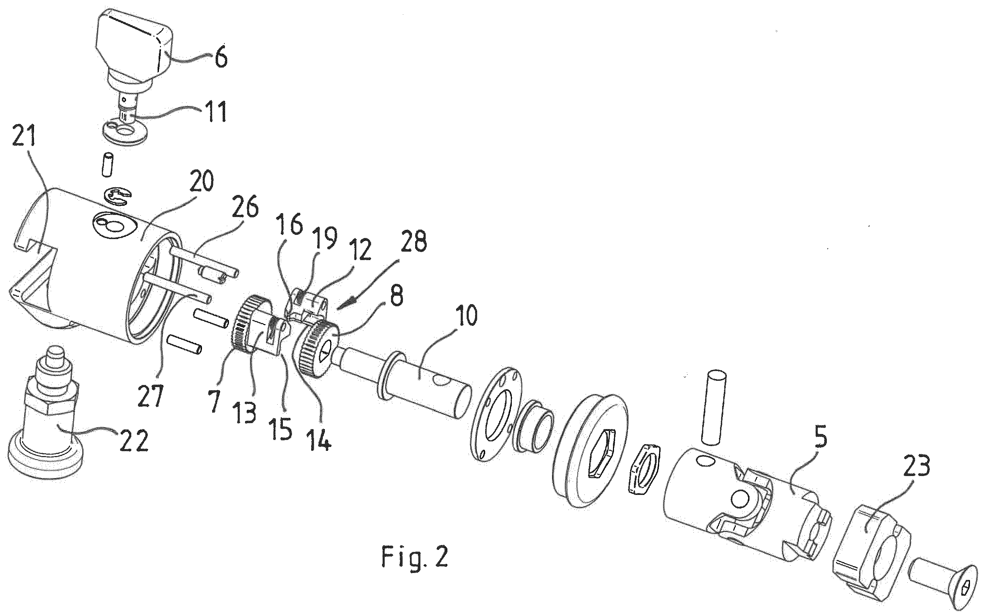

- Fig. 2: einer Explosionsdarstellung der Fußrotationsvorrichtung,
- Fig. 3 - 6: geschnittene Ansichten des Zahngesperres in unterschiedlichen Schaltpositionen,
- Fig. 7-10: geschnittene Draufsichten auf die Zahngesperre gemäß den Fig. 3 - 6,

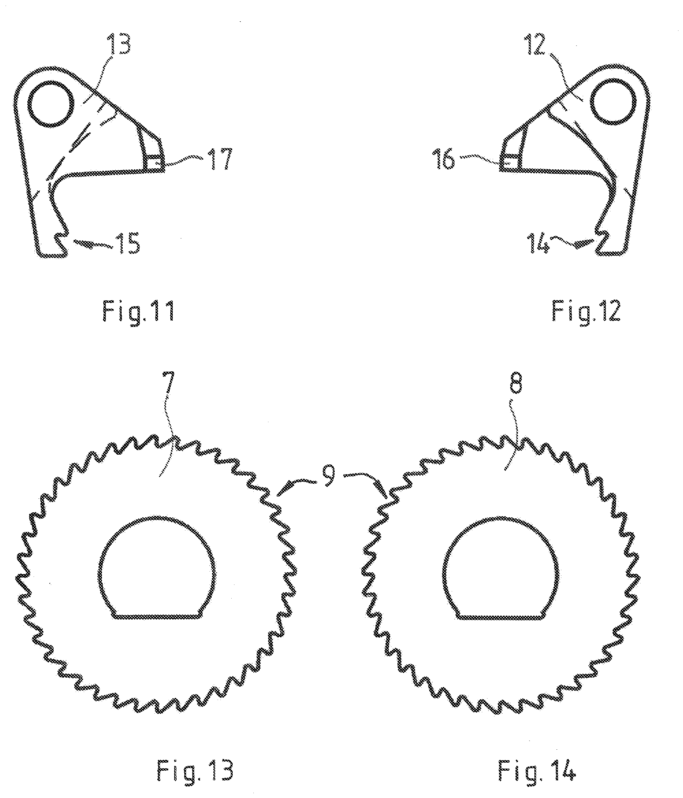

- Fig. 11: einen vergrößert dargestellten linken Sperrhebel
- Fig. 12: einen vergrößert dargestellten rechten Sperrhebel,
- Fig. 13: ein vergrößert dargestelltes linkes Sperrzahnrad, und
- Fig. 14: ein vergrößert dargestelltes rechtes Sperrzahnrad.

Die Fußrotation 1 einer Fußrotationsvorrichtung eines OP-Tisches ist an einem verschwenkbaren Ausleger angeordnet, an den sie über ein Profilrohr 25 höhenverstellbar festgelegt ist. Sie weist weiterhin ein Handrad 24 auf, um eine Fußaufnahmeschale 2 mit einem während einer OP darin gelagerten Patientenfuß bzw. das Bein eines Patienten in eine gestreckte Lage bringen zu können. Unterhalb der Fußaufnahmeschale 2 ist im fersenseitigen Bereich ein Befestigungsblock 3 angeordnet, in den sich ein Verriegelungsblock 23 des Rotationsblockes 4 erstreckt, an dem ein Fortsatz 5 angreift, der als Gelenkstück ausgebildet ist und sich in das Gehäuse 20 des Rotationsblockes 4 erstreckt und dort mit der darin gelagerten Welle 10 verbunden ist.

Der Rotationsblock 4 ist als Zahngesperre für zwei Drehrichtungen ausgebildet und weist hierzu zwei Sperrzahnräder 7; 8 mit entgegengesetzt zueinander ausgebildeten radialen Sperrkerben 9 auf, wobei die beiden Sperrzahnräder 7;8 beabstandet voneinander auf der gemeinsamen Welle 10 angeordnet sind, zwischen die sich ein Schaltnocken 11 des Betätigungsdrehgriffes 6 erstreckt, wobei der Schaltnocken 11 eine kreiszylinderartige Grundform aufweist und mit einer sich etwa über die Hälfte des Umfanges mit einer im Durchmesser verringerten Innenbahn ausgestattet ist und sich daran anschließenden, bis auf den größeren Durchmesser der Außenbahn geschwungenen Kurvenbahnen, denen sich über den restlichen Umfang die Außenbahn als letztes Teilstück der gesamten Nockenbahn 18 anschließt.

Oberhalb der beiden Sperrzahnräder 7; 8 sind auf parallel zur Welle 10 angeordneten Sperrhebelachsen 26; 27 zwei Sperrhebel 12; 13 angeordnet, die Sperrklinken 14; 15 aufweisen, die in die Sperrzahnräder 7; 8 eingreifen oder davon abgehoben positioniert werden können. Hierzu sind sie mit Steuernocken 16; 17 ausgestattet, die auf der Nockenbahn 18 des Schaltnockens 11 aufliegen, wobei die beiden Steuernocken 16; 17 sich gegenüberliegend, aber nicht radial, sondern von einer Mittelebene durch den Schaltnocken 11 versetzt auf der Nockenbahn 18 anliegen, sodass sie in Abhängigkeit vom Verdrehwinkel des Betätigungsdrehgriffes 6 entweder gleichzeitig auf der Innenbahn des Schaltnockens 11 oder auf dessen Außenbahn aufliegen oder aber entweder ein Steuernocken 16; 17 auf der Innenbahn und ein Steuernocken 17; 16 auf der Außenbahn der Nockenbahn 18, sodass insgesamt vier Schaltpositionen erzeugt sind.

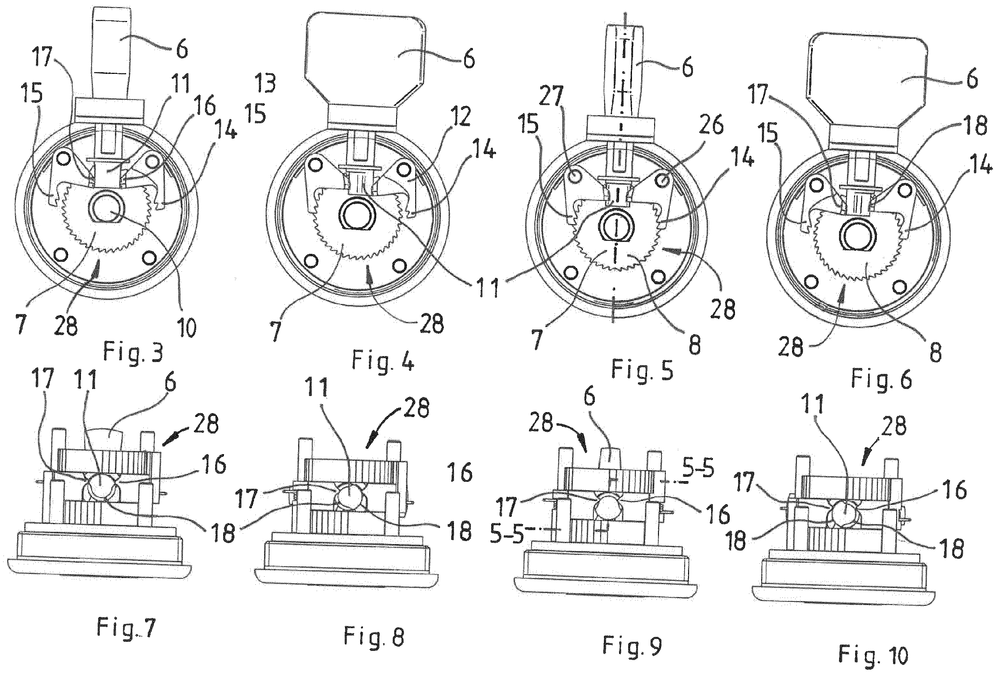

Die Figuren 3 und 7 zeigen eine Position der Schaltnocke 11 in zwei Ansichten, in der unter einem Verdrehwinkel des Betätigungsdrehgriffes 6 von 0° mit vollständigem beidseitigen Freilauf der Welle (10) beide Sperrklinken 14; 15 von den Sperrzahnrädern 7; 8 abgehoben sind. Die Figuren 5 und 9 zeigen eine Position der Schaltnocke 11 in zwei Ansichten bei vollständiger beidseitiger Sperrung der Welle bei 180° des Verdrehwinkels des Betätigungsdrehgriffes 6, in der die Steuernocken 16; 17 beide auf der Innenbahn der Nockenbahn 18 des Schaltnockens 11 aufliegen, sodass die Sperrhebel 12; 13 um die Sperrhebelachsen 26; 27 verschwenkt mit ihren Sperrklinken 14;15 in die Sperrzahnräder 7; 8 eingreifen und so eine beidseitige Drehverriegelung erzeugen.

Die Figuren 4 und 8 bzw. 6 und 10 stellen bei um 90° bzw. 270° gedrehtem Betätigungsdrehgriff 6 Schaltpositionen dar, bei denen entweder die Rechtsdrehung des Sperrzahnrades 7 oder die Linksdrehung des Sperrzahnrades 8 und damit jeweils der Welle 10 blockiert ist, wobei die jeweils anliegenden Sperrklinken 14; 15 bei einer entgegengesetzten Drehrichtung über die flacheren Flanken der Sperrkerben 9 kurzeitig entrastend hinweggeleiten können. Die Sperrklinken 14; 15 werden dabei über eine Feder 19 permanent in Richtung auf die Sperrzahnräder 7; 8 vorgespannt gehalten, sodass jederzeit ein Formschluss in die gesperrte Drehrichtung erfolgt.

## Patentansprüche

1. Fußrotationsvorrichtung eines OP-Tisches mit einer an einem verschwenkbaren Ausleger angeordneten Fußrotation (1) mit einer Fußaufnahmeschale (2) mit einem fersenseitigen Befestigungsblock (3) und einem sich davon in einen mit einer Arretiervorrichtung versehenden Rotationsblock (4) erstreckenden Fortsatz (5), wobei der Fortsatz (5) als Gelenkstück und die Arretiervorrichtung des Rotationsblocks (4) als Zahngesperre (28) für zwei Drehrichtungen ausgebildet ist und der Rotationsblock (4) einen Betätigungsdrehgriff (6) aufweist und in Abhängigkeit dessen Verdrehwinkels mindestens eine Drehrichtung gesperrt oder freigegeben ist, **dadurch gekennzeichnet, dass** im Rotationsblock (4) zwei Sperrzahnräder (7; 8) mit entgegengesetzt zueinander ausgebildeten radialen Sperrkerben (9) geringfügig voneinander beabstandet auf einer gemeinsamen Welle (10) angeordnet sind, die mit dem Fortsatz (5) verbunden ist, sich ein Schaltnocken (11) des Betätigungsdrehgriffes (6) zwischen die Sperrzahnräder (7; 8) erstreckt und zwei Sperrhebel (12; 13) mit Sperrklinken (14; 15) um zur Drehachse der Welle (10) parallele Sperrhebelachsen (26; 27) schwenkbar im Rotationsblock (4) gelagert sind, die Steuernocken (16;17) aufweisen, mit denen sie permanent auf einer Nockenbahn (18) des Schaltnocken (11) aufliegen und in Abhängigkeit dessen Verdrehwinkels eine oder beide Sperrklinken (14; 15) in ihr zugehöriges Sperrzahnrad (7; 8) eingreifen oder davon abgehoben sind.

2. Fußrotationsvorrichtung nach Anspruch 1, **dadurch gekennzeichnet, dass** sich der Schaltnocken (11) zwischen die Sperrhebel (12; 13) erstreckt, die Steuernocken (16; 17) aufweisen, die elastisch vorgespannt auf einer Nockenbahn (18) des Schaltnockens (11) aufliegen, der eine kreiszylinderartige Grundform aufweist, wobei sich die Nockenbahn (18) etwa über die Hälfte des Umfangs als Innenbahn aus einem kreiszylinderartigen Abschnitt mit kleinerem Durchmesser als dem der Grundform und sich daran anschließend bis auf den größeren Durchmesser geschwungenen Kurvenbahnen zusammensetzt sowie aus einem Teilstück mit dem Durchmesser der Grundform als Außenbahn.

3. Fußrotationsvorrichtung nach Anspruch 2, **dadurch gekennzeichnet, dass** die Steuernocken (16; 17) sich gegenüberliegend und von einer Mittelebene durch den Steuernocken (9) parallel seitlich versetzt an der Nockenbahn (18) anliegen und entweder gleichzeitig auf der Innenbahn oder auf der Außenbahn oder abwechselnd auf einer der beiden Bahnen aufliegen und vier Schaltpositionen mit 90° Winkelabstand zueinander erzeugt sind, mit vollständigem beidseitigen Freilauf der Welle (10) bei 0°, vollständiger beidseitiger Sperrung der Welle (10) bei 180° oder linksseitigen Freilauf mit rechtseitiger Sperrung bei 90° oder rechtseitigem Freilauf mit linksseitiger Sperrung bei 270°.

## Claims

1. Foot rotation device of an operating table, comprising a foot rotator (1) which is arranged on a pivotable boom and has a foot receiving shell (2) with a heel-side fastening block (3), and the rotation device comprising a projection (5) extending therefrom into a rotation block (4) provided with a locking device, the projection (5) being designed as a joint piece and the locking device of the rotation block (4) being designed as a ratchet mechanism (28) for two directions of rotation and the rotation block (4) having an actuating rotary handle (6) and at least one direction of rotation being blocked or released depending on the angle of rotation thereof, **characterized in that** two ratchet wheels (7; 8), having radial locking notches (9) formed opposite one another, are arranged in the rotation block (4) so as to be slightly spaced apart on a common shaft (10) which is connected to the projection (5), a switching cam (11) of the actuating rotary handle (6) extends between the ratchet wheels (7; 8), and two locking levers (12; 13) having locking pawls (14; 15) are mounted in the rotation block (4) so as to be pivotable about locking lever pins (26; 27) parallel to the axis of rotation of the shaft (10), which locking levers have control cams (16; 17) with which they permanently rest on a cam track (18) of the switching cam (11) and, depending on the angle of rotation thereof, one or both locking pawls (14; 15) engage in their associated ratchet wheel (7; 8) or are detached from it.

2. Foot rotation device according to claim 1, **characterized in that** the switching cam (11) extends between the locking levers (12; 13) which have control cams (16; 17) which rest in a resiliently preloaded manner on a cam track (18) of the switching cam (11), which has a circular-cylindrical basic shape, the cam track (18) being composed, over approximately half of the circumference, of a circular-cylindrical portion having a smaller diameter than that of the basic shape and, adjacent thereto, curved-in curved paths up to the larger diameter as the inner track and of a sub piece having the diameter of the basic shape as the outer track.

3. Foot rotation device according to claim 2, **characterized in that** the control cams (16; 17) rest on the cam path (18) opposite one another and laterally offset in parallel from a central plane by the control cam (9) and rest either simultaneously on the inner track or on the outer track or alternately on one of the two tracks, and four switching positions are generated with an angular distance of 90° from one another, with complete bilateral freewheeling of the shaft (10) at 0°, complete bilateral locking of the shaft (10) at 180°, or left-hand freewheeling with right-hand locking at 90° or right-hand freewheeling with left-hand locking at 270°.

## Revendications

1. Dispositif de rotation pour pied d'une table d'opération, comportant une rotation pour pied (1) disposée sur un bras pivotant et comportant une coque de réception pour pied (2) comportant un bloc de fixation (3) côté talon et un prolongement (5) s'étendant depuis celui-ci dans un bloc de rotation (4) pourvu d'un dispositif d'arrêt, dans lequel le prolongement (5) est réalisé sous forme de pièce articulée et le dispositif d'arrêt du bloc de rotation (4) est réalisé sous forme de mécanisme de blocage à dents (28) pour deux sens de rotation et le bloc de rotation (4) présente une poignée rotative d'actionnement (6) et au moins un sens de rotation est bloqué ou libéré en fonction de l'angle de rotation de ladite poignée rotative d'actionnement, **caractérisé en ce que**, dans le bloc de rotation (4), deux roues dentées de blocage (7 ; 8) comportant des encoches de blocage (9) radiales réalisées en sens inverse l'une de l'autre sont disposées à faible distance l'une de l'autre sur un arbre (10) commun qui est relié au prolongement (5), une came de commutation (11) de la poignée rotative d'actionnement (6) s'étend entre les roues dentées de blocage (7 ; 8) et deux leviers de blocage (12 ; 13) comportant des cliquets de blocage (14 ; 15) sont montés dans le bloc de rotation (4) de manière à pouvoir pivoter autour d'axes de levier de blocage (26 ; 27) parallèles à l'axe de rotation de l'arbre (10), lesquels présentent des cames de commande (16 ; 17) par lesquelles ils reposent en permanence sur une piste de came (18) de la came de commutation (11) et, en fonction de l'angle de rotation de celle-ci, un cliquet de blocage (14 ; 15) ou les deux viennent en prise dans leur roue dentée de blocage (7 ; 8) associée ou en sont retirés.

2. Dispositif de rotation pour pied selon la revendication 1, **caractérisé en ce que** la came de commutation (11) s'étend entre les leviers de blocage (12 ; 13) qui présentent des cames de commande (16 ; 17) qui reposent de manière précontrainte élastiquement sur une piste de came (18) de la came de commutation (11) qui présente une forme de base de type cylindre circulaire, dans lequel la piste de came (18) se compose, sur environ la moitié de la circonférence, d'une section de type cylindre circulaire en tant que piste intérieure, comportant un diamètre inférieur à celui de la forme de base, et de pistes courbes incurvées se raccordant à ladite piste intérieure jusqu'au plus grand diamètre, ainsi que d'un tronçon en tant que piste extérieure, comportant le diamètre de la forme de base.

3. Dispositif de rotation pour pied selon la revendication 2, **caractérisé en ce que** les cames de commande (16 ; 17) s'appuient sur la piste de came (18) de manière à être opposées l'une à l'autre et décalées latéralement et parallèlement par rapport à un plan médian passant par la came de commande (9) et s'appuient soit simultanément sur la piste intérieure, soit sur la piste extérieure, soit alternativement sur l'une des deux pistes et quatre positions de commutation sont générées avec un écart angulaire de 90° entre elles, avec une roue libre complète des deux côtés de l'arbre (10) à 0°, un blocage complet des deux côtés de l'arbre (10) à 180° ou une roue libre côté gauche avec un blocage côté droit à 90° ou une roue libre côté droit avec un blocage côté gauche à 270°.
